(19) Europäisches Patentamt
European Patent Office
Office européen des brevets

(11) Veröffentlichungsnummer: **0 309 652 B1**

# (12) EUROPÄISCHE PATENTSCHRIFT

(45) Veröffentlichungstag der Patentschrift: **09.10.91**

(51) Int. Cl.$^5$: **A61K 7/13**

(21) Anmeldenummer: **88109511.1**

(22) Anmeldetag: **15.06.88**

(54) **Haarfärbemittel.**

(30) Priorität: **18.09.87 DE 3731396**
**11.02.88 DE 3804221**

(43) Veröffentlichungstag der Anmeldung:
**05.04.89 Patentblatt 89/14**

(45) Bekanntmachung des Hinweises auf die Patenterteilung:
**09.10.91 Patentblatt 91/41**

(84) Benannte Vertragsstaaten:
**AT BE CH DE ES FR GB GR IT LI LU NL SE**

(56) Entgegenhaltungen:
**EP-A- 0 136 730**
**DE-A- 2 934 331**
**DE-B- 1 949 750**

**C. ZVIAK: "THE SCIENCE OF HAIR CARE"**

(73) Patentinhaber: **RÜTGERSWERKE AKTIENGESELLSCHAFT**
**Mainzer Landstrasse 217**
**W-6000 Frankfurt am Main 1(DE)**

(72) Erfinder: **Orth, Winfried, Dr.**
**Am Schachtelgraben 28**
**W-6733 Hassloch/Pfalz(DE)**
Erfinder: **Weiss, Wolfgang, Dr.**
**Kelterweg 3**
**W-6803 Neckarhausen(DE)**
Erfinder: **Kleffner, Hans Werner, Dr.**
**Panoramastrasse 13**
**W-6719 Battenberg(DE)**

**EP 0 309 652 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services

**Beschreibung**

Die Erfindung betrifft Haarfärbemittel auf der Basis von Oxidationsfarbstoffen in Kombination mit einschlägigen Entwicklern. Die Färbung der Haare erfolgt dabei durch Reaktion der Entwicklersubstanzen mit sogenannten Kupplersubstanzen oder Nuanceuren in alkalischem Medium in Gegenwart eines geeigneten Oxidationsmittels. Durch die im allgemeinen dabei entstehenden intensiven Farben mit sehr guten Echtheitseigenschaften und durch die große Variationsbreite der Farbtöne spielen diese Oxidationsfarben eine bedeutende Rolle in der Haarkosmetik.

Als Kuppler- oder Nuanceurkomponenten kennt man m-Phenylendiaminderivate, Phenole, Naphthole oder Resorcinderivate. Da diese Produkte alle toxikologisch und dermatologisch nicht unbedenklich sind, versucht man, auf die unbedenklicheren heterocyclischen Verbindungen überzugehen.

So kennt man die Verwendung als Kupplersubstanz von
2,3- bzw. 2,5-Diaminopyridin aus DE-PS 11 42 045,
2,5-Diaminopyridin aus DD-PS 57 402,
Bis-aminopyridinen aus EP 0 008 079 B1,
Dihydroxypyridinen aus US-PS 1 571 570,
Hydroxy- und Alkoxypyridinaminen aus FR-PS 1 397 551 und FR-PS 1 398 193,
Pyridylaminobenzolen und Bispyridylaminen aus FR-PS 1 401 469,
Nitropyridinaminen aus EP-A 0 137 524
oder
Dinitropyridinaminen aus EP-A 0 193 656.

Trotz dieser bereits bestehenden Vielfalt von Haarfärbemitteln besteht ein weiterer Bedarf, teils weil manche dieser Kupplungskomponenten nicht ausreichend beständig gegen Luftsauerstoff sind oder nicht mit Tensiden der verschiedenen Gruppen kombinierbar sind, teils um die Palette von Farbnuancen noch besser mit intensiven Farben mit sehr guten Echtheitseigenschaften abdecken zu können. Insbesondere besteht ein Bedarf nach Rottönen, da man aus toxikologischen Gründen gerne auf das bislang hierfür verwendete Nitro-p-phenylendiamin verzichten möchte.

Es bestand daher die Aufgabe, Haarfärbemittel auf Basis von Oxidationsfarbstoffen zu entwickeln, die intensive Farben mit sehr guten Echtheitseigenschaften vor allem im Bereich der Rottöne ergeben und deren Kuppler- oder Nuanceurkomponenten gegen Luftsauerstoff weitgehend beständig sind, so daß sie auch in geringen Mengen in neutraler oder Salzform eingesetzt werden können.

Die Lösung der Aufgabe erfolgt durch Bereitstellung der Mittel gemäß der Ansprüche 1 und 2 sowie der speziellen Verbindungen gemäß der Ansprüche 3 bis 6, die intensive Haarfarben ergeben.

Es wurde gefunden, daß Aminopyrrolderivate der allgemeinen Formel I

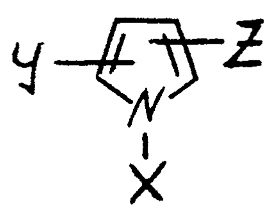

(I)

in der X Wasserstoff, eine Methyl- oder Ethylgruppe darstellt, Y in Position 4 oder 5 steht und Wasserstoff, eine Amino-, Hydroxy- oder Methylgruppe, eine Alkoxygruppe mit 1 bis 3 C-Atomen oder einen zweiten Rest Z darstellt und Z in Position 2 oder 3 steht und eine Gruppe der allgemeinen Formel II

-($C_nH_{2n}$)-NH-R (II)

bedeutet, worin n eine Zahl von 1 bis 3 bedeutet und R einen unsubstituierten oder an beliebiger Stelle amin-, methoxy- oder ethoxy- oder amin- und methoxy- oder ethoxy-substituierten N-Methylpyrrol- oder N-Ethylpyrrol-, Pyrrol-, Pyridin-, Piperidin-, Pyrimidin- oder Morpholinrest darstellt, in Kombination mit einschlägigen Entwicklerkomponenten und Oxidationsmitteln gut Kuppler- oder Nuanceurkomponenten darstellen, die die obengenannten Anforderungen erfüllen, auch wenn sie in geringen Mengen eingesetzt werden. Insbesondere überrascht, daß diese Aminopyrrolderivate bevorzugt zu brillanten Rottönen führen.

Durch Variation der Substituenten am Pyrrolring und an der Aminogruppe der erfindungsgemäß eingesetzten Verbindungen lassen sich verschiedene Farbvarianten erzielen. Es ist somit mit diesen Mitteln

möglich, durch Abmischung verschiedener Nuanceurkomponenten nahezu alle Farbvarianten mit einem Oxidationsfarbensystem einzustellen. Die erfindungsgemäßen Haarfärbemittel stellen somit eine Bereicherung auf dem Gebiet der Haarkosmetik dar.

Erfindungsgemäß in Oxidationsfarbstoffsystemen einzusetzende Kupplerkomponenten sind Pyrrol-, N-Methylpyrrol- oder N-Ethyl-pyrrolderivate, die in Position 2 oder 3 am Pyrrolring eine Gruppe der allgemeinen Formel $-(C_nH_{2n})-NH-R$ besitzen. Dabei kann der restliche Pyrrolring unsubstituiert sein oder in Position 4 oder 5 durch eine Methyl-, Methoxy-, Ethoxy-, Propoxy-, Hydroxy- oder Aminogruppe oder durch eine zweite Alkylaminogruppe der allgemeinen Formel $-(C_nH_{2n})-NH-R$ substituiert sein.

Die sich daran anschließende Aminogruppe ist mit einem unsubstituierten oder an beliebiger Stelle substituierten Pyrrol-, N-Methylpyrrol-, N-Ethylpyrrol-, Pyridin-, Piperidin-, Pyrimidin- oder Morpholinrest substituiert.

Die Substituenten dieser Heterocyclen können sowohl eine Aminogruppe oder eine Methoxy- oder Ethoxygruppe als auch eine Aminogruppe und eine Methoxy- oder Ethoxygruppe sein.

Die erfindungsgemäßen Aminopyrrolderivate können in an sich bekannter Weise durch Reduktion der entsprechenden Nitropyrrolderivate hergestellt werden. Beispiele für entsprechende Nitropyrrolderivate wie auch Beispiele für deren Herstellungsverfahren sind aus DE-A 33 34 029 bekannt.

Die erfindungsgemäßen Kupplungskomponenten können jeweils alleine oder zur Einstellung gewünschter Farbnuancen in Abmischung miteinander oder mit anderen an sich bekannten Nuanceur- oder Kupplerkomponenten eingesetzt werden.

Als Beispiel für in den erfindungsgemäßen Haarfärbemitteln einzusetzende Entwicklerkomponenten sind primäre aromatische Amine mit einer weiteren in p-Stellung befindlichen funktionellen Gruppe wie p-Phenylendiamin,
Alkylamino-p-phenylendiamine,
p-Toluylendiamin,
p-Aminophenol,
N-Methyl-p-phenylendiamin,
N,N-Dimethyl-p-phenylendiamin,
N,N-Diethyl-2-methyl-p-phenylendiamin,
N-Ethyl-N-hydroxyethyl-p-phenylendiamin,
Chlor-p-phenylendiamin,
N,N-Bis-hydroxyethylamino-p-phenylendiamin,
Methoxy-p-phenylendiamin,
2,6-Dichlor-p-phenylendiamin,
2-Chlor-6-brom-p-phenylendiamin,
2-Chlor-6-methyl-p-phenylendiamin,
6-Methoxy-3-methyl-p-phenylendiamin,
andere Verbindungen der genannten Art, die weiterhin eine oder mehrere funktionelle Gruppen wie OH-Gruppen, $NH_2$-Gruppen, NHR-Gruppen, $NR_2$-Gruppen, wobei R einen Alkyl- oder Hydroxyalkylrest mit 1 - 4 Kohlenstoffatomen darstellt, ferner heterocyclische Hydrazonderivate wie
1-Methylpyrrolidon-(2)-hydrazon,
4-Aminopyrazolonderivate wie 4-Amino-1-phenyl-3-carbamoylpyrazolon-5,
N-Butyl-N-sulfobutyl-p-phenylendiamin,
Tetraaminopyrimidine wie
2,4,5,6-Tetraaminopyrimidin,
4,5-Diamino-2,6-bismethylaminopyridin,
2,5-Diamino-4-diethylamino-6-methylaminopyrimidin,
2,4,5-Triamino-6-dimethylaminopyrimidin,
2,4,5-Triamino-6-piperidino-pyrimidin,
2,4,5-Triamino-6-anilino-pyrimidin,
2,4,5-Triamino-6-morpholinopyrimidin,
2,4,5-Triamino-6-$\beta$-hydroxy-ethylaminopyrimidin,
aber auch Pyridinderivate wie z. B. 2,5-Diaminopyridin oder 2,5-Diamino-4-methylpyridin anzuführen.

Die oxidative Kupplung, d. h. die Entwicklung der Färbung könnte grundsätzlich, wie bei anderen Oxidationshaarfarbstoffen auch durch Luftsauerstoff erfolgen. Jedoch ist für die praktische Anwendung die Reaktionsgeschwindigkeit zu gering und die Farbentwicklung auf dem Haar zu langsam. Daher werden bevorzugt chemische Oxidationsmittel eingesetzt. Als solche kommen insbesondere Wasserstoffperoxid oder dessen Anlagerungsprodukte an Harnstoff, Melamin und Natriumborat sowie Gemische aus derartigen Wasserstoffperoxidanlagerungsverbindungen mit Kaliumperoxidisulfat in Betracht.

Die erfindungsgemäßen Haarfärbemittel, die die Kupplungs- und Entwicklerkomponente enthalten, werden für den Einsatz in entsprechende kosmetische Zubereitungen wie Cremes, Emulsionen, Gele oder auch einfach Lösungen eingearbeitet. Dazu ist es mitunter notwendig, die Lösungen bis zu 100 °C zu erhitzen, um die Komponenten in Lösung zu bringen, gegebenenfalls unter Mithilfe eines Lösungsvermittlers. Dabei liegen in gebrauchsfähigen Produkten die Konzentrationen an Kupplungskomponenten zwischen 0,01 - 2 Gew.-% und an Entwicklerkomponente zwischen 0,1 - 5 Gew.-%. Zur Herstellung der kosmetischen Zubereitung werden die Komponenten mit den für derartige Zubereitungen üblichen weiteren Bestandteilen gemischt.

Als solche zusätzlichen Bestandteile sind zum Beispiel Ammoniumhydroxid, Netz- oder Emulgiermittel vom anionischen oder nichtionogenen Typ wie Alkylbenzolsulfonate, Fettalkoholsulfonate, Fettalkoholethersulfate, Aminoxide, Alkylsulfonate, Fettsäurealkanolamide, Alkylphenoloxalkyllate und Anlagerungsprodukte von Ethylenoxid an Fettalkohol, Reduktionsmitteln, wie Natriumsulfit, Natriumdithionit, Thioglykolsäure oder Ascorbinsäure, Verdickungsmittel wie Methylcellulose, höhere Fettalkohole, Fettsäuren, ferner Parfümöle und Haarpflegemittel wie Pantothensäure und Cholesterin zu nennen.

Kurz vor der Anwendung werden diese Haarfärbemittel mit einer Lösung eines der genannten Oxidationsmittel wie üblich gemischt und die so erhaltene Mischung auf das Haar aufgetragen. Die Anwendungstemperaturen bewegen sich dabei im Bereich von 30 bis 40 °C. Nach einer Einwirkungsdauer von ca. 30 Minuten wird das Haarfärbemittel vom zu färbenden Haar durch Spülen entfernt. Hernach wird das Haar mit einem milden Shampoo nachgewaschen und getrocknet.

Beispiele

Beispiele 1 bis 5

Mit Haarfärbemitteln folgender Grundzusammensetzung werden Färbeversuche durchgeführt:

2 Gew.-% 30%ige Fettsäureaminoxidlösung,
0,5 Gew.-% Natriumdithionit,
10 Gew.-% 25%iges Ammoniumhydroxid,
0,5 Gew.-% erfindungsgemäßer Kupplungskomponente,
1 Gew.-% Phenylendiamin,
86 Gew.-% Wasser.

100 ml der Haarfärbemittel werden mit 10 ml Wasserstoffperoxidlösung (6 %) gemischt. In diese Mischungen werden Haarsträhnen aus Naturhaar eingetaucht und die Farblösung läßt man 30 Minuten bei 35 °C einwirken. Anschließend werden die Strähnen gut ausgespült, getrocknet und hinsichtlich ihrer Färbung beurteilt.

Beispiele 1

2-(5′-1′-Methylpyrrol-methylenamino)-5-methyl-pyrrol

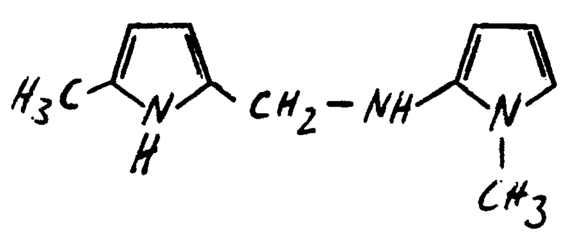

Farbe: rotbraun

Beispiel 2

2-(5′-Amino-2′-methoxy-6′-ethylenaminopyridin)-1H-pyrrol

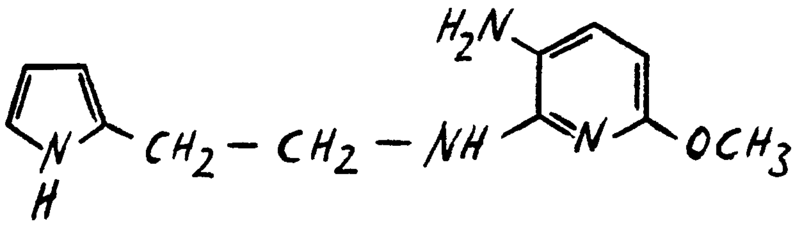

Farbe: rot-rotviolett

Beispiel 3

2-(3′-Amino-6′-ethylenaminopyridin)-1H-pyrrol

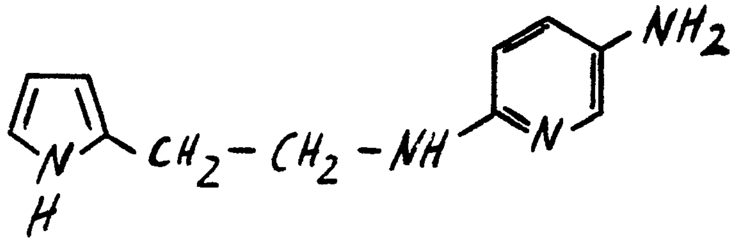

Farbe: rot-rotviolett

Beispiel 4

2-(5′-Amino-2′-methoxy-6′-ethylenaminopyridin)-1-methyl-1H-pyrrol

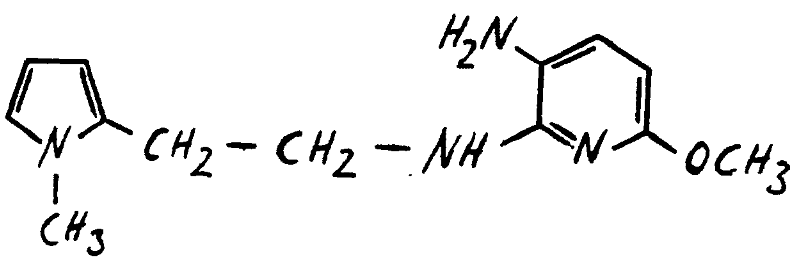

Farbe: himbeerrot

Beispiel 5

2-(3′-Amino-6′-ethylenaminopyridin)-1-methyl-1H-pyrrol

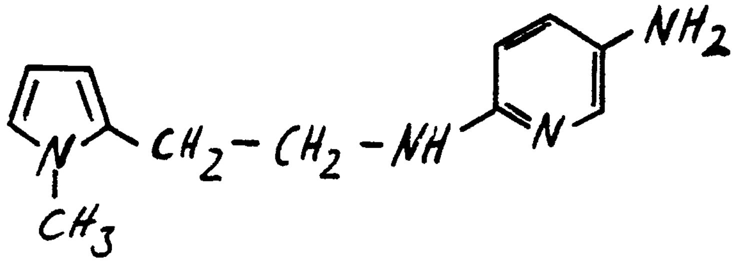

Farbe: himbeerrot

Beispiel 6

2-(5′-Amino-2′-methoxy-6′-ethylenaminopyridin)-1H-1-methyl-pyrrol

69 g (0,25 Mol) 2-(2′-Methoxy-5′-nitro-6′-ethylenaminopyridin)-1H-1-methyl-pyrrol werden mit 25 g Raney-Nickel als Katalysator 20 h bei 65 °C und 6-7 bar $H_2$-Druck in 1000 ml Methanol als Lösungsmittel reduziert.
Das Reaktionsgemisch wird mit 3000 ml Wasser versetzt, das ausgefallene Amin abgenutscht, mehrmals mit Wasser nachgewaschen und bei 60 °C im Vakuum getrocknet.

Das Rohprodukt zeigt eine violette Farbe. Diese rührt von einem roten Nebenprodukt her, das nur in sehr geringer Menge vorhanden ist.

Eine Reinigung durch Umkristallisation ist nicht möglich.
Ausbeute: 27 g = 44 %
Fp: 93-94 °C
Elementaranalyse :
Ber. C 63.4 H 7.3 N 22.7 %
Gef. C 62.8 H 7.3 N 22.6 %

Beispiel 7

2-(5′-Amino-2′-methoxy-6′-ethylenamino)-1H-pyrrol

Analog zu Beispiel 6 werden 65,5 g 2-(2′-methoxy-5′-nitro-6′-ethylenamino)-1H-pyrrol reduziert.
Erhalten werden 25 g eines leicht violetten Produkts.
Fp: 98-99 °C

Beispiel 8

2-(3′-Amino-6′-ethylenaminopyridin)-1H-pyrrol

Analog zu Beispiel 6 werden 52,25 g 2-(3′-Nitro-6′-ethylenaminopyridin)-1H-pyrrol reduziert.
Erhalten werden 19 g einer leicht violetten Substanz.
Fp: 23-24 °C

Beispiel 9

2(3′-Amino-6′-ethylenaminopyridin)-1H-1-methyl-pyrrol

Analog zu Beispiel 6 werden 60,75 g 2-(3′-Nitro-6′-ethylenaminopyridin)-1H-1-methyl-pyrrol reduziert
Erhalten werden 24 g einer violetten, öligen Substanz.
Fp des Hydrochlorids:

## Patentansprüche

1. Haarfärbemittel auf der Basis von Oxidationshaarfarben, die Entwickler-, Kupplungs-und Oxidationskomponenten enthalten, **dadurch gekennzeichnet,** daß sie als Kupplungskomponenten ein oder mehrere Aminopyrrolderivate der allgemeinen Formel I

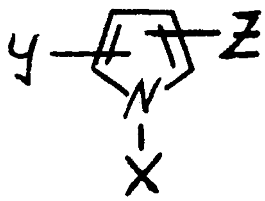

(I)

enthalten, in der X Wasserstoff, eine Methyl- oder Ethylgruppe darstellt, Y in Position 4 oder 5 steht und Wasserstoff, eine Amino-, Hydroxy- oder Methylgruppe, eine Alkoxygruppe mit 1 bis 3 C-Atomen oder einen zweiten Rest Z darstellt und Z in Position 2 oder 3 steht und eine Gruppe der allgemeinen Formel II

$-(C_nH_{2n})-NH-R$ (II)

bedeutet, worin n eine Zahl von 1 bis 3 bedeutet und R einen unsubstituierten oder an beliebiger Stelle amin-, methoxy- oder ethoxy- oder amin- und methoxy- oder ethoxy-substituierten N-Methylpyrrol- oder N-Ethylpyrrol-, Pyrrol-, Pyridin, Piperidin-, Pyrimidin- oder Morpholinrest darstellt.

2. Haarfärbemittel nach Anspruch 1, **dadurch gekennzeichnet,** daß sie neben den Alkylaminopyrrolderivaten noch andere Kupplungskomponenten enthalten.

3. 2-(5′-Amino-2′-methoxy-6′-ethylenaminopyridin)-1H-pyrrol.

4. 2-(5′-Amino-2′-methoxy-6′-ethylenaminopyridin)-1H-1-methyl-pyrrol.

5. 2-(3′-Amino-6′-ethylenaminopyridin)-1H-pyrrol.

6. 2-(3′-Amino-6′-ethylenaminopyridin)-1H-1-methyl-pyrrol.

## Claims

1. Hair dyes based on oxidation hair tints which contain developer, coupling and oxidation components, characterized in that they contain one or more aminopyrrole derivatives of the general formula I

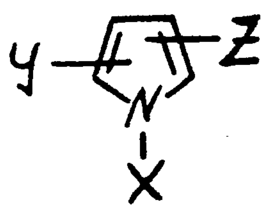

(I)

as coupling components, in which X represents hydrogen, a methyl or ethyl group, Y is in position 4 or 5 and represents hydrogen, an amino, hydroxy or methyl group, an alkoxy group with from 1 to 3 C atoms or a second residue Z and Z is in position 2 or 3 and represents a group of the general formula II

$-(C_nH_{2n})-NH-R$ (II)

in which n signifies a number of from 1 to 3 and R represents an N-methyl pyrrole or N-ethyl pyrrole, pyrrole, pyridine, piperidine, pyrimidine or morpholino radical which may be unsubstituted or substituted with an amino, methoxy or ethoxy or amino and methoxy or ethoxy group at any position.

2. Hair dyes according to Claim 1, characterized in that in addition to the alkylaminopyrrole derivatives they also contain other coupling components.

3. 2- (5'-amino-2'-methoxy-6'-ethyleneaminopyridine)-1H-pyrrole.

4. 2- (5'-amino-2'-methoxy-6'-ethyleneaminopyridine)-1H-1-methyl-pyrrole.

5. 2- (3'-amino-6'-ethyleneaminopyridine)-1H-pyrrole.

6. 2- (3'-amino-6'-ethyleneaminopyridine)-1H-1-methyl-pyrrole.

**Revendications**

1. Teintures capillaires à base de colorants capillaires d'oxydation contenant des composants développateurs, de copulation et d'oxydation, caractérisées en ce qu'elles contiennent en tant que composants de copulation un ou plusieurs dérivés d'aminopyrrole de formule générale I

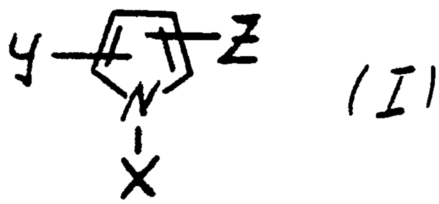

dans laquelle X représente l'hydrogène, un groupe méthyle ou éthyle, Y se trouve en position 4 ou 5 et représente l'hydrogène, un groupe amino, hydroxy ou méthyle, un groupe alcoxy avec 1 à 3 atomes de C ou un deuxième radical Z et Z se trouve en position 2 ou 3 et signifie un groupe de formule générale II

$-(C_nH_{2n})-NH-R$ (II)

où n signifie un nombre de 1 à 3 et R représente un radical N-méthylpyrrole ou N-éthylpyrrole, pyrrole, pyridine, pipéridine, pyrimidine ou morpholine non substitué ou substitué en un endroit quelconque par une amine, un méthoxy ou un éthoxy ou par une amine et un méthoxy ou un éthoxy.

2. Teintures capillaires selon la revendication 1, caractérisées en ce qu'elles contiennent encore d'autres composants de copulation à côté des dérivés d'alkylaminopyrrole.

3. 2-(5'-amino-2'-méthoxy-6'-éthylèneaminopyridine)-1H-pyrrole.

4. 2-(5'-amino-2'-méthoxy-6'-éthylèneaminopyridine)-1H-1-méthylpyrrole.

5. 2-(3'-amino-6'-éthylèneaminopyridine)-1H-pyrrole.

6. 2-(3'-amino-6'-éthylèneaminopyridine)-1H-1-méthylpyrrole.